# EUROPÄISCHE PATENTANMELDUNG

(11) **EP 2 290 348 A2**
(43) Veröffentlichungstag der Anmeldung: **02.03.2011**
(21) Anmeldenummer: 10008888.9
(22) Anmeldetag: 26.08.2010
(51) Int. Cl.: G01N 11/04

(54) **Messvorrichtung für Fluide**

(30) Priorität: 26.08.2009 DE 202009011492 U
(71) Anmelder: Vogt, Joachim, 89079 Ulm-Unterweiler (DE); Kalunka, Daniel, 73760 Ostfildern (DE)
(72) Erfinder: Vogt, Joachim, 89079 Ulm-Unterweiler (DE); Kalunka, Daniel, 73760 Ostfildern (DE)
(74) Vertreter: Fiener, Josef

(57) **Zusammenfassung**

Zur Schaffung einer einfachen und kostengünstigen Messvorrichtung (10) für Fluide, insbesondere für Betriebs- und/ oder Schmiermittel in einem Antrieb (1) oder einem Gerät, wird ein Füllstandsmessrohr (22) vorgeschlagen, in dem ein zugeordnetes Viskositätsmessrohr oder zugeordneter Viskositatsmessrohrabschnitt integriert ist Bevorzugt ist das Füllstandsmessrohr (22) einem Peilstabführungsrohr (18) für einen Peilstab (12) zugeordnet, insbesondere einem Ölmessstab.

## Beschreibung

Die Erfindung betrifft eine Messvorrichtung für Fluide mit den oberbegrifflichen Merkmalen des Anspruchs 1.

In Geräten und Antriebseinheiten (z. B. Motoren, Getrieben usw.), insbesondere mit beweglichen Teilen, die mit verschiedenen Betriebs- und Schmiermitteln befüllt sind, ist eine Überwachung der Fluid-/ bzw. Betriebsmittelqualität wichtig für die Lebensdauer. Die Fluidqualität muss sich innerhalb festgelegter Grenzen bewegen, um eine verschleißarme und somit wartungsarme Funktion der Geräte bzw. Antriebe zu gewährleisten. Daneben muss sichergestellt werden, dass für die Fluidmenge tolerierbare Minimal- und Maximalgrenzwert weder unter- noch überschritten werden.

Herkömmlicherweise wird die Überwachung der Fluidqualität sowie der Fluidmenge getrennt und durch den Einsatz aufwändiger Sensoren durchgeführt. Ein Qualitätsmerkmal für die Flüssigkeit ist beispielsweise deren Viskosität. Um eine Messung der Viskosität einer Flüssigkeit durchzuführen, um Erkenntnisse über die Flüssigkeitsqualität zu gewinnen, schlägt die DE 42 10 845 A1 eine Vorrichtung vor, die ein komplexes System mit mehreren Viskosimeterröhren, einem Ventilsystem und einem Temperaturbad aufweist. Das System führt eine Messung von entnommenen Proben durch, eine direkte Anbindung an die zu messende Flüssigkeit bzw. den Flüssigkeitskreislauf ist nicht vorgesehen. Der Aufbau des Systems ist darüber hinaus äußerst komplex und damit zum einen in der Erstellung sehr kostenintensiv und darüber hinaus stark wartungs- und überwachungsbedürftig. Eine Nachrüstung ist nur schwer durchführbar und mit Umbauten an den entsprechenden Geräten oder Antriebseinheiten verbunden.

Die Über- oder Unterschreitung einer Fluidmenge wird über Füllstandssensoren oder - schalter gemessen. Bei dieser Messmethode ist z. B. auch die konstruktive Integration der Sensoren oder Schalter in das Gerät nötig, was ebenfalls mit hohem Einbau- und nachfolgenden Wartungsaufwand einhergeht.

Aufgabe der vorliegenden Erfindung ist es, eine Messvorrichtung für Fluide zur Verfügung zu stellen, die auf einfache und kostengünstige Art und Weise erstellt werden kann und sich problemlos und in kompakter Weise in einen herzustellenden oder bestehenden Antrieb oder ein herzustellendes oder bestehendes Gerät einbauen lässt.

Diese Aufgabe wird gelöst durch eine Messvorrichtung für Fluide mit den Merkmalen des Anspruchs 1. Vorteilhafte Weiterbildungen der Erfindung sind Gegenstand der abhängigen Ansprüche.

Die erfindungsgemäße Messvorrichtung eignet sich für Fluide und hierbei insbesondere für Betriebs- und/ oder Schmiermitteln in einem Antrieb oder einem Gerät. Nachfolgend bezieht sich die Beschreibung nur noch auf Betriebsmittel, wobei diese dann Schmiermittel sowie alle übrigen Fluide in einem Antrieb oder einem Gerät umfassen, deren optimaler Füllstand und optimale Viskosität für den reibungslosen und dauerhaft wartungsarmen Betrieb eines Antriebs oder Gerätes von Bedeutung sind. Mit der erfindungsgemäßen Messvorrichtung wird die Möglichkeit geschaffen, auf einfache Art und Weise sowohl den Füllstand eines Betriebsmittels als auch dessen Viskosität zu messen. Hierzu ist die Messvorrichtung gekennzeichnet durch ein Füllstandsmessrohr, in dem ein Viskositätsmessrohr oder ein zugeordneter Viskositätsmessrohrabschnitt integriert ist. Die Messvorrichtung ist dabei besonders kompakt ausgeführt, da das Viskositätsmessrohr oder der Viskositätsmessrohrabschnitt in dem Füllstandsmessrohr integriert, d.h. es umschließt das Füllstandsmessrohr das Viskositätsmessrohr vollständig oder teilweise, bzw. das Viskositätsmessrohr bildet einen Teil des Füllstandsmessrohres, z. B. durch eine parallele Anordnung. Das Viskositätsmessrohr oder der Viskositätsmessrohrabschnitt kann dabei auch konzentrisch im Füllstandsmessrohr oder dessen inneren Umfang kontaktierend angeordnet werden. Desgleichen besteht die Möglichkeit, dass das Füllstandsmessrohr konzentrisch im Viskositätsmessrohr oder dem Viskositätsmessrohrabschnitt bzw. an dessen inneren Umfang kontaktierend angeordnet ist. Als weitere Ausgestaltungsmöglichkeit umfasst die Erfindung auch die unmittelbar benachbarte Anordnung von Viskositätsmessrohr oder Viskositätsmessrohrabschnitt und Füllstandsmessrohr, wobei sich die Teile der Messvorrichtung zumindest teilweise berühren oder über die Mantelflächen der Messrohre miteinander verbunden sind.

Das Viskositätsmessrohr oder der Viskositätsmessrohrabschnitt erlaubt eine Sichtprüfung der Viskosität des Betriebsmittels durch erfahrene Messpersonen. Um jedoch besonders präzise und reproduzierbare Messungen der Viskosität im Messrohr durchführen zu können, ist es günstig, wenn die Messvorrichtung eine Messeinheit für die Viskosität des Fluids aufweist. Hierfür eignen sich am Messrohr unmittelbar angeordnete Sensoren für die Fluidparameter, wie Temperatursensoren oder Sensoren für die Fließgeschwindigkeit oder die Dichte des Fluids, z. B. Photozellen, die eine genaue Bestimmung von für die Berechnung der Viskosität eines Fluids heranziehbaren Werten ermöglichen.

Um eine genaue Messung der Viskosität durchführen zu können, ist es wichtig, eine bestimmte Menge an Fluid messen zu können. Um diese Menge zur Verfügung stellen zu können, ist es vorteilhaft, wenn das Viskositätsmessrohr oder ein zugeordneter Viskositätsmessrohrabschnitt absperrbar ausgebildet ist. Die Absperrung kann dabei insbesondere über beabstandet angeordnete, gesondert, beispielsweise zeitlich versetzt schaltbare Absperrventile erfolgen. Daneben besteht auch die Möglichkeit entsprechend wirkende Klappen, Schieber oder Absperrhähne in dem Viskositätsmessrohr oder dem Viskositätsmessrohrabschnitt anzuordnen.

Eine besonders kompakte Bauweise der Messvorrichtung kann dadurch erreicht werden, dass dem Füllstandsmessrohr ein Führungsrohr für einen (ÖI-)Peilstab zugeordnet ist und das Viskositätsmessrohr in das Führungsrohr integriert ist. Neben dem Viskositätsmessrohr kann auch die Messeinheit für die Viskosität des Fluids in das Führungsrohr integriert vorliegen. Mit einem einzigen Bauteil, nämlich dem Führungsrohr werden so Messungen sowohl des Flüssigkeitspegels, d.h. der im Gerät oder im Antrieb vorhandenen Flüssigkeitsmenge, als auch der Flüssigkeitsviskosität und damit indirekt der Flüssigkeitsqualität, möglich. Das diese Messmittel umfassende Bauteil bleibt dabei in seinen Abmessungen kompakt und kann auf einfache Art und Weise in ein Gerät oder einen Antrieb bei der Herstellung verbaut oder auch nachträglich in bestehende Systeme integriert werden, insbesondere als Ölmessstab-Einheit bei einem Motor oder Getriebe.

Als vorteilhaft wird im Zusammenhang mit der vorliegenden Erfindung erachtet, wenn mit dem Betriebsmittelkreislauf und hierbei insbesondere mit dem Ölkreislauf in Verbindung stehende Leitungen oder Kanäle, und hierbei beispielsweise Zuführ- oder Rücklaufleitungen, als Viskositätsmessrohr ausgebildet ist/sind. Insbesondere bei der Qualitäts- und Mengen- bzw. Füllstandsüberwachung von Öl eignen sich Ölkühlerleitung oder Ölrücklaufleitung für die zusätzliche Funktionalisierung als Viskositätsmessrohr.

Neben der Integration in bestehende Rohre oder Leitungen sieht eine vorteilhafte Weiterbildung der Erfindung vor, dass am Antrieb, dem Füllstandsmessrohr oder dem Führungsrohr Anbindungs- oder Schnittstellen für das Viskositätsmessrohr vorgesehen sind, so dass entsprechende in einem Gerät oder Antrieb bereits vorhandene Rohre, Leitungen oder Kanäle für die Nachrüstung einer zusätzlichen Viskositätsmessfunktion herangezogen werden können. Dadurch lässt sich ein vereinfachter Aufbau und eine kostengünstigere Nachrüstung realisieren.

Neben der integrierten Bauweise der Messvorrichtung erweist es sich auch als günstig, wenn das Viskositätsmessrohr eine gesonderte Leitung aufweist. Durch diese Ausführungsform wird eine Flexibilität beim Einbau bzw. bei der Nachrüstung erreicht, da das Viskositätsmessrohr bzw. dessen Anordnung am Gerät oder Antrieb nicht auf die Position des Füllstandsmessrohres beschränkt bleibt, sondern in dessen Umfeld frei angeordnet werden kann. Dies ist insbesondere dann vorteilhaft, wenn das Viskositätsmessrohr und/oder die Messeinheit von dem Füllstandsmessrohr oder von dem Führungsrohr thermisch oder schwingungstechnisch entkoppelt sein sollen.

Um auf die in der Viskositäts- bzw. Füllstandsmessung ermittelten Werte reagieren zu können, und um beispielsweise die Funktionen des Antriebs bei Unter- oder Überfüllung oder bei mangelhafter Fluidqualität im Interesse einer Verschleißminderung anpassen zu können, erweist es sich als vorteilhaft, wenn die Messeinheit mit wenigstens einem Steuergerät für das Gerät oder den Antrieb verbunden ist, sodass hierüber eine Leistungsminderung bzw. rechtzeitige Abschaltung bei Gefahr von Beschädigungen erfolgen kann. Um die Möglichkeit zu schaffen, bereits aufbereitete, beispielsweise entsprechend gefilterte Werte oder Wertebereiche für die ermittelten Messwerte an das Steuergerät oder eine Ausgabeeinheit weitergeben zu können, erweist es sich als günstig, wenn schon der Messeinheit wenigstens eine entsprechende Auswerteeinheit zugeordnet ist.

Eine weitere günstige Ausführungsform der erfindungsgemäßen Messvorrichtung sieht vor, dass wenigstens ein zusätzlicher Sensor, wenigstens ein zusätzliches Ventil und/oder wenigstens ein zusätzlicher Schalter in der Messeinheit vorgesehen ist, um hier weitere Messvorrichtungen ankoppeln bzw. ansteuern zu können und/oder um das Wertespektrum hinsichtlich der Messwertmenge und der Messwertbreite bzw. -vielfalt erweitern zu können.

In diesem Zusammenhang und um die Einsetzbarkeit und Kompatibilität der erfindungsgemäßen Messvorrichtung zu verbessern und zu erweitern, wird es als vorteilhaft angesehen, wenn die Messeinheit für die Erfassung von Werten aus wenigstens einem Steuergerät oder aus wenigstens einer weiteren Erfassungs- und Auswerteeinheit ausgebildet ist. Durch die Erfassung von Werten des Steuergerätes besteht die Möglichkeit, in Rückkopplung mit der Antriebs- oder Gerätesteuerung die Messwertdichte oder -art zu vergrößern bzw. anzupassen, um hier eine noch präzisere Steuerung durchführen zu können. Mit den Werten aus weiteren Erfassungs- und Auswerteeinheiten kann eine noch präzisere Überwachung der Funktionen des Antriebs oder Gerätes erreicht und die Steuerung dadurch weiter verbessert werden. Durch die Integration in ein Gerät bzw. eine Messeinheit wird eine kompaktere Bauweise der Messvorrichtung erreicht und zudem die Nachrüstung wie auch der Einbau wesentlich vereinfacht. Es muss nur eine einzige zusätzliche Einheit vorgesehen werden, um eine Vielzahl von Werten, auch aus bestehenden Erfassungseinheiten, wie den in einem Antrieb serienmäßig enthaltenen Sensoren und Sonden, zusätzlich aus- und für eine Optimierung verwerten zu können.

Um eine schnelle Erfassung und kurzfristige Auswertung durchführen zu können, ist es günstig, wenn die Messeinheit und/oder die Erfassungs- und Auswerteeinheit wenigstens eine Anzeige aufweist, auf der Rohdaten und/oder bereits verarbeitete Daten angezeigt werden können. Hier besteht erfindungsgemäß die Möglichkeit, die Anzeige direkt oder indirekt mit der Messeinheit und/oder der Erfassungs- und Auswerteeinheit zu koppeln, d.h. direkt an der Messeinheit und/oder der Erfassungs- und Auswerteeinheit die Werte anzuzeigen oder aber eine entsprechende Anzeige in eine Anzeigetafel oder ein Überwachungsdisplay zu integrieren.

Weitere Vorteile, Merkmale und Besonderheiten der Erfindung ergeben sich aus der nachfolgenden Beschreibung bevorzugter, jedoch nicht beschränkender Ausführungsformen der Erfindung anhand der schematischen Zeichnungen. Hierbei zeigen:
- Fig. 1: eine schematische Darstellung einer Ausführungsform der Messvorrichtung; und
- Fig. 2: eine schematische Darstellung einer bevorzugten Ausführungsform der erfindungsgemäßen Messvorrichtung, jeweils in seitlicher Schnittdarstellung.

Die vorliegende Erfindung soll beispielhaft anhand der Viskosität- und Füllstandsüberwachung in einer Antriebseinheit 1 erläutert werden. Hier soll das Motoröl 21 als Betriebsstoff überwacht werden. Gemessen wird das Motoröl 21 in ölführenden oder ölnahen Leitungen, also in Leitungen oder Kanälen, die mit dem Ölkreislauf direkt oder indirekt in Verbindung stehen. Hierbei ist es auch möglich, vorhandene Anbindungs- oder Schnittstellen, wie z. B. Blindstopfen, Rohrverschraubungen o. dgl. an den entsprechenden Antriebseinheiten 1, Leitungen oder Kanäle für die Anbindung bzw. Integration einer Viskosität- und Füllstandsüberwachung zu nutzen.

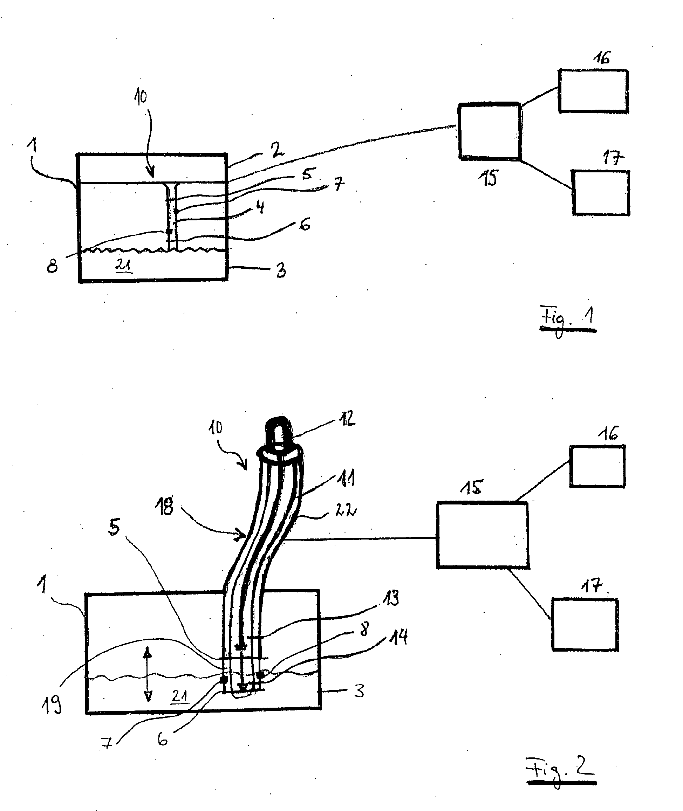

Fig. 1 zeigt eine mögliche Ausführungsform der Messvorrichtung 10 und hierbei insbesondere deren für die Viskositätsmessung ausgelegten Teil. Der Zylinderkopf 2 einer Antriebseinheit 1 wird über eine Ölpumpe (nicht dargestellt) fortlaufend mit Motoröl 21 versorgt und weist zur Füllstandmessung eine in den Rücklaufkanal 4 integrierte Messvorrichtung 10 auf. Über solche Rücklaufkanäle 4 läuft das Motoröl 21 zurück in die Ölwanne 3. Über Ventile 5, 6 kann dieser Rücklauf kurzzeitig unterbrochen werden. Um nun die Viskosität des Motoröls 21 zu messen, wird ein erstes, im Ausführungsbeispiel der Fig. 1 unteres Ventil 6 geschlossen, so dass sich der Rücklaufkanal 4 mit Motoröl 21 füllt. Sobald dieser voll ist, wird das obere Ventil 5 geschlossen. Ein Temperatursensor 7 misst die Temperatur des Motoröls 21. Sobald diese für eine Messung geeignet ist, wird das untere Ventil 6 geöffnet. Über einen im Ausführungsbeispiel optischen Sensor 8 wird die Abfließgeschwindigkeit bzw. die Leerlaufzeit des Rücklaufkanals 4 gemessen. Über diese Geschwindigkeit bzw. Leerlaufzeit kann unter Berücksichtigung der Temperatur auf die Ölviskosität rückgeschlossen werden. Die ölführende bzw. ölnahen Leitungen können dabei auch außen an der Antriebseinheit 1 verlaufen, wobei sich hierzu insbesondere Ölkühlerleitungen oder Rücklaufschläuche eignen.

Daneben bleibt die Erfindung nicht auf die genannten Kanäle und Leitungen beschränkt, sondern bietet die Möglichkeit, entsprechende Messvorrichtungen 10 vorhandenen Ölkanälen zuzuordnen bzw. in diese zu integrieren. Die Auswertung der in den Sensoren 7, 8 erfassten Werte erfolgt in diesen jeweils zugeordneten Steuergeräten 16, 17 oder einem zentralen Steuergerät 15. Vorteil in der dargestellten Messvorrichtung 10 ist, dass schon vorhandene Komponenten der Antriebseinheit 1 für die Messungen verwendet werden können, sodass eine Integration der Messvorrichtung 10 hier besonders einfach, schnell und kostengünstig durchgeführt werden kann.

Fig. 2 zeigt eine Ausführungsform der Messvorrichtung 10 mit in der Füllstandsmessung integrierter Viskositätsmessung. Das Motoröl 21, dessen Zustand und Menge erfasst werden soll, befindet sich wiederum in der Ölwanne 3 einer Antriebseinheit 1. In der dargestellten Ausführungsform ist die Messvorrichtung 10 für Füllstands- und Viskositätsmessung in ein Peilstabführungsrohr 11 (sog. Ölmessstab) integriert, d.h. das Peilstabführungsrohr 18 ist doppelwandig aufgebaut und umfasst ein inneres Führungsrohr 11 für den Peilstab 12 sowie ein äußeres, konzentrisch mit dem Peilstabführungsrohr 18 angeordnetes Füllstandsmessrohr 22. Die Überwachung des Füllstands erfolgt über den im Füllstandsmessrohr 22 eingebauten Maximumsensor 13 bzw. den Minimumsensor 14. Der Peilstab 12 zur manuellen Überprüfung des Ölstandes befindet sich im Führungsrohr 11. Ändert sich nun das Fluidniveau, kann über den Minimumsensor 14 eine Unterfüllung und über den Maximumsensor 13 eine Überfüllung detektiert werden.

Die Auswertung der Daten erfolgt z. B. in dem zentralen Steuergerät 15 oder in den jeweiligen Sensoren 13, 14, 7, 8 zugeordneten Steuergeräten 16, 17. Es kann auch auf weitere Messgrößen dieser Steuergeräte 15, 16, 17 zugegriffen werden. Zusätzlich zur Füllstandsmessung wird die oben bereits beschriebene Viskositätsmessung des Motoröls 21 durchgeführt. Hierzu werden entsprechende Ventile 5, 6 in das Füllstandsmessrohr 22 eingebaut und für die Viskositätsmessung nach Befüllung des zwischen den Ventilen 5, 6 liegenden Rohrabschnittes 19 geschlossen. Zusätzlichen zu den Ventilen 5, 6 sind ein Temperatursensor 7 und ein optischer Sensor 8 in den Rohrabschnitt 19 integriert. Zur Viskositätsmessung erfolgt eine Befüllung des Rohrabschnittes 19 über den in der Antriebseinheit 1 schon vorhandenen Unterdruck oder über Druckunterschiede innerhalb oder an der Antriebseinheit 1. In einer einfacheren Ausführungsform kann statt des Maximumsensors 13 bzw. des Minimumsensors 14 der Peilstab 12 und das Peilstabführungsrohr 11 zur Messung des Füllstandstandes bzw. der Viskosität verwendet werden. Die entsprechenden Ventile 5, 6 werden dann direkt im unteren Endbereich des Peilstabführungsrohres 11 angeordnet. Selbstverständlich besteht auch die Möglichkeit, dass entlang dem Peilstabführungsrohr 18 (bzw. in Fig. 1 dem Rücklaufkanal 4) ein paralleler Rohrabschnitt 19 zur Durchführung der Viskositätsmessung vorgesehen wird, der dann unmittelbar benachbart oder entkoppelt vom Peilstabführungsrohr 18 bzw. dem Rücklaufkanal 4 in der Messvorrichtung 10 angeordnet werden kann.

Neben einer optischen Messung der Viskosität kann bei entsprechender Ausführung der Sensoren 7, 8, 13, 14 selbstverständlich auch eine kapazitive Messmethode eingesetzt werden. Daneben kann die Messung über die Erfassung und Auswertung von Mikrowellen-, Ultraschall-, Infrarot- oder sonstigen allgemeinen Frequenzsignalen oder in sonstiger geeigneter Weise erfolgen. Vorteil der erfindungsgemäßen und in den Fig. 1 und 2 dargestellten Messvorrichtungen 10 ist, dass die verwendeten Führungsrohre 4, 11, 18 bzw. Rohrabschnitte 19 mit integrierter Messsensorik an oder zwischen einzelnen Fahrzeugen einfach ausgetauscht werden können und sich somit zur Nachrüstung bzw. temporären Ausrüstung, beispielsweise bei Testläufen o. dgl. eignen.

### Bezeichnungsliste

- 1 =: Antriebseinheit
- 2 =: Kopf
- 3 =: Ölwanne
- 4 =: Rücklaufkanal
- 5 =: oberes Ventil
- 6 =: unteres Ventil
- 7 =: Temperatursensor
- 8 =: optischer Sensor
- 10 =: Messvorrichtung
- 11 =: inneres Führungsrohr
- 12 =: Peilstab
- 13 =: Maximumsensor
- 14 =: Minimumsensor
- 15, 16, 17 =: Steuergerät
- 18 =: Peilstabführungsrohr
- 19 =: Rohrabschnitt
- 21 =: Motoröl
- 22 =: Füllstandsmessrohr

## Patentansprüche

1. Messvorrichtung (10) für Fluide, insbesondere für Betriebs- und/ oder Schmiermittel in einem Antrieb (1) oder einem Gerät, **gekennzeichnet durch**
ein Füllstandsmessrohr (22), in dem ein zugeordnetes Viskositätsmessrohr oder zugeordneter Viskositätsmessrohrabschnitt integriert ist.

2. Messvorrichtung nach Anspruch 1, **dadurch gekennzeichnet, dass**
das Viskositätsmessrohr bzw. der Viskositätsmessrohrabschnitt und das Füllstandsmessrohr (22) unmittelbar benachbart zueinander angeordnet sind.

3. Messvorrichtung nach Anspruch 1 oder 2, **dadurch gekennzeichnet, dass**
das Viskositätsmessrohr bzw. der Viskositätsmessrohrabschnitt eine Messeinheit für die Viskosität des Fluids aufweist.

4. Messvorrichtung nach Anspruch 3, **dadurch gekennzeichnet, dass**
die Messeinheit Sensoren für die Fluidparameter, insbesondere wenigstens einen Temperatursensor (7) und einen Sensor (8) für die Fließeigenschaften des Fluids umfasst. _

5. Messvorrichtung nach einem der Ansprüche 1 bis 4, **dadurch gekennzeichnet, dass**
das Viskositätsmessrohr oder ein zugeordneter Viskositätsmessrohrabschnitt absperrbar ausgebildet ist, insbesondere beabstandet angeordnete Absperrventile (5, 6) aufweist.

6. Messvorrichtung nach einem der Ansprüche 1 bis 5, **dadurch gekennzeichnet, dass**
dem Füllstandsmessrohr (22) ein Peilstabführungsrohr (18) für einen Peilstab (12) zugeordnet ist und das Viskositätsmessrohr bzw. der Viskositätsmessrohrabschnitt und/oder die Messeinheit in das Peilstabführungsrohr (18) integriert ist.

7. Messvorrichtung nach einem der Ansprüche 1 bis 6, **dadurch gekennzeichnet, dass**
mit dem Betriebs- und/ oder Schmiermittelkreislauf in Verbindung stehende Leitungen oder Kanäle, insbesondere wenigstens eine Ölkühlerleitung oder eine Ölrücklaufleitung (4) als Viskositätsmessrohr bzw. Viskositätsmessrohrabschnitt ausgebildet ist/sind.

8. Messvorrichtung nach einem der Ansprüche 1 bis 7, **dadurch gekennzeichnet, dass**
am Antrieb (1) oder am Füllstandsmessrohr (22) Anbindungs- oder Schnittstellen für die Messvorrichtung (10) oder das Viskositätsmessrohr bzw den Viskositätsmessrohrabschnitt vorgesehen sind.

9. Messvorrichtung nach einem der Ansprüche 1 bis 8, **dadurch gekennzeichnet, dass**
das Viskositätsmessrohr bzw. der Viskositätsmessrohrabschnitt eine gesonderte Leitung aufweist.

10. Messvorrichtung nach einem der Ansprüche 1 bis 9, **dadurch gekennzeichnet, dass**
das Viskositätsmessrohr bzw. der Viskositätsmessrohrabschnitt und/oder die Messeinheit von dem Füllstandsmessrohr (22) entkoppelt angeordnet ist/sind.

11. Messvorrichtung nach einem der Ansprüche 3 bis 10, **dadurch gekennzeichnet, dass**
die Messeinheit mit wenigstens einem Steuergerät (15, 16, 17) verbunden ist.

12. Messvorrichtung nach einem der Ansprüche 3 bis 11, **dadurch gekennzeichnet, dass**
der Messeinheit wenigstens eine Auswerteeinheit zugeordnet ist.

13. Messvorrichtung nach einem der Ansprüche 3 bis 12, **dadurch gekennzeichnet, dass**
die Messeinheit wenigstens einen zusätzlichen Sensor und/oder wenigstens ein zusätzliches Ventil und/oder wenigstens einen zusätzlichen Schalter aufweist.

14. Messvorrichtung nach einem der Ansprüche 3 bis 13, **dadurch gekennzeichnet, dass**
die Messeinheit für die Erfassung von Messwerte wenigstens eines Steuergerätes (15, 16, 17) oder wenigstens einer weiteren Erfassungs- und Auswerteeinheit ausgebildet ist.

15. Messvorrichtung nach einem der Ansprüche 3 bis 14, **dadurch gekennzeichnet, dass**
die Messeinheit wenigstens eine Anzeige aufweist, wobei eine direkte oder indirekte Kopplung der Messeinheit mit der Anzeige vorgesehen ist.
